# EUROPÄISCHE PATENTSCHRIFT

(11) **EP 1 849 441 B1**
(45) Veröffentlichungstag und Bekanntmachung des Hinweises auf die Patenterteilung: **17.12.2008**
(21) Anmeldenummer: 07008109.6
(22) Anmeldetag: 20.04.2007
(51) Int. Cl.: A61F 5/01

(54) **Hüftdysplasie-Bandagenorthese**
Orthotic bandage for hip dysplasia
Orthèse de bandage pour dysplasie de la hanche

(30) Priorität: 24.04.2006 DE 102006018881
(43) Veröffentlichungstag der Anmeldung: 31.10.2007
(73) Patentinhaber: Shuku, Aida, 90429 Nürnberg (DE)
(72) Erfinder: Mittelmeier, Heinz Prof.em.Dr.med.Dr.h.c., 6373 Ennetbürgen/NW (CH); Graf, Reinhard Primarius Prof.Dr.med., 8852 Stolzalpe (AT)
(74) Vertreter: Kramer - Barske - Schmidtchen

(56) Entgegenhaltungen:
- DE-A1- 2 018 240
- DE-C1- 3 714 083
- DE-C1- 3 737 546

## Beschreibung

Die Erfindung betrifft eine Hüftdysplasie-Bandagenorthese (Hüftdysplasie-Bandage) zur funktionellen Frühbehandlung der kindlichen Hüftdysplasie. Das Dokument DE-A-2 018 240 stellt der nächste Stand der Technik dar.

Die Hüftdysplasie ist das häufigste angeborene Skelettleiden und führt unbehandelt zu schweren Gelenkverformungen und Verrenkungen mit sekundärer Arthrose. Aufgrund der modernen Frühdiagnostik mit Ultraschall (R. GRAF, seit 1980) ist es möglich, mit einem unschädlichen bildgebenden Verfahren die Fälle früh zu erfassen und einer erfolgreichen konservativen Frühbehandlung mit Hilfe von sogenannten Spreizhosen bzw. Dysplasie-Bandagen zuzuführen. Hiermit werden die nach der Geburt durch die Streckstellung der Beine entstehenden, gegen das Pfannendach gerichteten muskulären Schubkräfte in mehr transversaler Richtung gegen den Pfannengrund umstellt und eine formative Nachreifung des Gelenkes mit guter knöcherner Formsicherung durch das Pfannendach ermöglicht. Mit einer Beugestellung über 90° lassen sich hiermit sogar kaudal gerichtete Schubkräfte erzeugen, welche eine Wiedereinrenkung teilweise oder ganz verrenkter Gelenke ermöglichen. Wichtig ist dabei die Gewährleistung eines guten dynamischen Bewegungsspieles der Hüftgelenke in der therapeutischen Beuge-Spreizstellung, und dass bei Fällen mit bereits erfolgter Dislokation die Hüftgelenke wesentlich über 90° angebeugt werden können sowie eine nur mäßige Abspreizung mit Vorhaltesicherung besteht, um Durchblutungsschäden und nachfolgende Wachstumsstörungen der Hüftköpfe zu vermeiden.

Eine Hüftdysplasie-Bandage zur funktionellen Frühbehandlung der kindlichen Hüftdysplasie ist aus der DE 37 37 546 C1 bekannt. Diese Hüftdysplasie-Bandage enthält ein steifes Beckenteil, an dem unter 45°-Abspreizstellungen zu einer Mittelachse lang gestreckte Beinteile derart angeschlossen sind, dass sie einen Winkel von 90° zwischen sich einschließen. Diese Seitenteile sind zum Umgreifen der Oberschenkel des Kindes in Beugestellung vorgesehen und sind an ihren freien Enden an einem gürtelartigen Rumpfgurt, der am Beckenteil angebracht ist, zu befestigen. Bei dieser Hüftdysplasie-Bandage werden die beiden Oberschenkel des Kindes und damit die Hüftgelenke somit nicht durch eine einheitliche Spreizvorrichtung gleichartig in die therapeutische Beuge-Spreizstellung gebracht, sondern durch aufgespaltene schwalbenschwanzartig gestaltete, mit dem Beckenteil fest verbundene Beinteile als sogenannte Oberschenkelhalteschlaufen getrennt in etwa 45° Beugestellung über der Horizontalen gehalten, so dass auch ausreichend kaudal gerichtete Repositionskräfte entstehen. Die gemäßigte Abspreizposition wird dabei durch eingebaute, vom Becken bis zum Oberschenkelbereich durchlaufende Vorhaltekeile gesichert.

Diese Spreizbandage hat sich in der Praxis im Laufe der Jahre in zahlreichen Fällen insgesamt gut bewährt, obgleich sie jedoch nicht genau graduell den optimalen biomechanischen Erforderungen des einzelnen Hüftbefundes des Patienten angepasst werden kann und außerdem keine Anpassungsmöglichkeit an das gerade im frühkindlichen Alter rasche Größenwachstum der Säuglinge vorsieht, so dass bei einem längeren Therapieverlauf eine nochmalige, mit erneuten Kosten verbundene größere Bandage erforderlich ist.

Da die Hüftgelenke eines Patienten oft in unterschiedlicher Weise von der Dysplasie betroffen sind, ist darüber hinaus eine seitendifferente und unterschiedliche Hüftbeugestellung wünschenswert. Dies hat zur Folge, dass mit der bekannten Bandage von der Dysplasie nicht betroffene Hüftseiten ggf. unnötig mitbehandelt werden, während verhältnismäßig stark oder übermäßig betroffene Teile zu wenig oder ungenügend behandelt werden.

Davon ausgehend ist es Aufgabe der vorliegenden Erfindung, eine Hüftdysplasie-Bandage vorzusehen, welche diese Mängel behebt und somit eine Spreizbandage mit definierter, individueller, seitendifferenter, exakter, gradueller Einstellbarkeit der therapeutisch erforderlichen Hüftbeugung (mit angemessener Abspreiz-Vorhaltestellung) sowie eine Anpassungsfähigkeit der Bandage an das Größenwachstum des Kindes schafft, was zusammen erhebliche Behandlungsvorteile und eine Kosteneinsparung ergibt.

Diese Aufgabe wird durch eine Hüftdysplasie-Bandage mit den Merkmalen des Anspruchs 1 gelöst. Bevorzugte Ausführungsformen sind in den abhängigen Ansprüchen angegeben.

Es ist bevorzugt, dass die Kunststofffolien durchsichtige Polyethylenfolien sind.

Es ist weiter bevorzugt, dass die Löcher im Beckenteil annähernd in der sagittalen Projektion der Hüftgelenke des Säuglings liegen.

Es ist weiter bevorzugt, dass die Befestigunsvorrichtung einen Stift und/oder einen Niet aufweist.

Es ist weiter bevorzugt, dass die Befestigungseinrichtung eine Kompressionsschraube enthält, die mit einer selbsthaltenden Mutter festziehbar ist, wobei an der Spitze der Kompressionsschraube vorzugsweise eine Quetschsicherung gegen eine völlige Mutterablösung vorgesehen ist.

Es ist weiter bevorzugt, dass zwischen die Schraube und die sich daran anschließenden Becken- und Beinteile Beilagsscheiben eingebracht sind.

Es ist weiter bevorzugt, dass die Kompressionsschraube aus Metall oder Kunststoff ist.

Es ist weiter bevorzugt, dass das Befestigungsmittel ein in den Langlöchern führbarer Clip ist.

Es ist weiter bevorzugt, dass ein zusätzliches Sicherungsmittel zur sicheren Lagepositionierung der Beinteile zu dem Beckenteil vorgesehen ist.

Es ist weiter bevorzugt, dass das zusätzliche Sicherungsmittel zusätzliche Löcher in dem Beckenteil und den Beinteile mit einem zweiten Clip ist.

Es ist weiter bevorzugt, dass das zusätzliche Sicherungsmittel ein Klettmittel ist.

Es ist weiter bevorzugt, dass das Beckenteil eine formstabile, querverlaufende Bügelschiene aufweist, die mit dem Beckenteil fest verbunden ist.

Es ist weiter bevorzugt, dass die Bügelschiene aus steifem Kunststoff oder Metall ist.

Es ist weiter bevorzugt, dass die Bügelschiene einen Mittenwinkel von 110° einnimmt.

Es ist weiter bevorzugt, dass zwischen das planare Folienteil und das sich daran anschließende Folienteil beiderseits Stützkeile mit einem Vorhaltewinkel von 35° eingeschoben und verstellbar befestigt sind.

Es ist weiter bevorzugt, dass die Stützkeile zur Erweiterung des Beckenteils zur Wachstumsanpassung in mehreren seitlichen Stellungen mit dem Folienteil verbindbar sind.

Es ist weiter bevorzugt, dass die Verschlusseinrichtung einen Klettverschluss enthält.

Es ist weiter bevorzugt, dass der Klettverschluss an dem Rumpfgurt einen Klettverschluss, der an den Beinteilen jeweils vorgesehen ist, aufnehmen kann.

Es ist weiter bevorzugt, dass das Beckenteil und die Beinteile mit einem flexiblen, dehnbaren, der Gesamtform der Bandage entsprechenden, abnehmbaren Textilüberzug versehen sind, welcher leicht abnehmbar am Beckenteil befestigt ist.

Es ist weiter bevorzugt, dass am Beckenteil und den Beinteilen im Bereich der Gelenkachsen beiderseits eine Winkelskala zur exakten Einstellung der Abspreizstellung der Beinteile gegenüber dem Beckenteil aufgedruckt ist.

Das grundlegende neue Konstruktionsprinzip besteht darin, dass - im wesentlichen Unterschied zu einer herkömmlichen einheitlichen Hüftbandage mit zusammenhängenden Becken-Beinteilen und damit unverstellbarer Hüftgelenksposition - die neue Bandage aus einem Beckenteil und zwei separaten Beinteilen besteht, welche beiderseits im projizierten Hüftgelenksbereich des Beckenteils durch z.B. eine entsprechende Scharnierachse mittels vorzugsweise einer Kompressionsschraube in unterschiedlicher Abspreizstellung der Beinteile und damit variabler Hüftbeugestellung ein- und festgestellt werden können. Insbesondere können die Beinteile der erfindungsgemäßen Bandage gegenüber dem Beckenteil in einer Verstellposition zumindest entweder verdreht und/oder translatorisch verschoben werden, um ihre Position bezüglich dem Beckenteil an das Ausmaß der Hüftdysplasie bzw. die Größe des Kindes anzupassen. Nach dem Einstellvorgang in der gewünschten Verstellposition können die Beinteile und das Beckenteil zueinander durch die Befestigungseinrichtung in der eingestellten Position derart festgelegt werden, dass sie sich bei der Anwendung nicht mehr relativ zueinander bewegen können.

Die Form des Beckenteiles der Bandage soll vorzugsweise dem Umriss des kindlichen Beckens mit den Weichteilen entsprechen und von der Rumpftaille bis zu den Gesäßfalten hinunterreichen, dort entsprechend den Gesäßbacken beiderseits verrundet sein und auch seitlich den Hüftbeugebereich abdecken. Nach einer bevorzugten Ausführungsform besteht das Beckenteil in dieser Form aus zwei gleichartigen halbsteifen durchsichtigen Kunststoff-Folien (vorzugsweise Polyäthylen) von 1-2 mm Dicke, welche im oberen Bereich und mittlings miteinander vernäht oder verbunden werden, so dass sie sich gegenseitig verstärken. Im latero-kaudalen (unteren) Bereich bleiben sie dagegen unverbunden, so dass hier der obere Bereich der Beinteile der Bandage zwischen sie eingeschoben werden kann und von ihnen geführt wird.

Nach einer bevorzugten Ausführungsform ist das Beckenteil jedoch nicht plan gestaltet, sondern weist in der Mitte eine vorzugsweise leicht verrundete Aufbiegung der beiden Seitenteile nach vorne auf, welche vorzugsweise je etwa 35° beträgt und den eingeschobenen Beinteilen die therapeutisch wünschenswerte Beugestellung der Hüftgelenke in gemäßigter Abspreizung mit Vorhaltesicherung verleiht.

Dazu kann die Biegung der Beckenplatten bei der Herstellung kalt oder auch thermoplastisch erfolgen.

Durch die gegenseitige Befestigung der beiden das Beckenteil bildenden Schichten wird die Vorhaltestellung durch die Ausbildung des Beckenteils nach dem Laminarprinzip gesichert. Zur weiteren Sicherung der Vorhaltestellung kann vorzugsweise im oberen Bereich des Beckenteils, zusätzlich ein entsprechend abgewinkelter schmaler, streifenförmiger Querbügel aus steifem Kunststoff (z.B. PVC) oder eine dünne Metallschiene eingebaut werden, die die seitliche Aufbiegung des Beckenteils unterstützt und hält.

Im unteren, nicht verbundenen Teil der Beckenplatte weist dieselbe in bevorzugter Weise im Hüftgelenksbereich eine Scharnierlochperforation von z.B. etwa 3 bis 4 mm Durchmesser auf, ebenso wie die separaten Beinteile im oberen medialen Bereich, so dass nach dem Einschieben der Beinteile in das Beckenteil und Kongruenz der Löcher z.B. eine Kompressionsschraube als Scharnierachse mit selbsthaltender Mutter durchgesteckt und die Beinteile in der gewünschten variablen Abspreizstellung durch Festziehen der Achsschraube festgestellt werden können. Andere Mittel zum lösbaren aber lagefesten Fixieren der Beinteile und des Beckenteils zueinander sind ebenfalls denkbar. Beispielsweise kann die Anzahl und Gestalt der beidseitigen Löcher variiert werden oder es können andere lösbare und wieder arretierbare Befestigungsmittel, wie Haftbänder, vorgesehen werden.

Um eine Größenanpassung der Beinteile an das Wachstum des Kindes zu ermöglichen, ist nach einer besonders bevorzugten Ausführungsform die Lochperforation des Beckenteiles im Bereich der Hüftgelenke als ein schräg gestelltes, von medio-kranial nach latero-kaudal verlaufendes, ca. 3 cm langes Schlitzloch gestaltet werden, welches bei fortschreitendem Wachstum des Kindes eine Verschiebung der Schraubachse nach distal-lateral und damit eine Verlängerung der Beinteile ermöglicht. Alternativ oder zusätzlich kann auch die Perforation der Beinteile als solches Schlitz- oder Langloch mit entsprechender Ausrichtung gestaltet sein.

Zur Erleichterung der Einstellbarkeit zur Größenanpassung sind vorteilhafter Weise auf dem Beckenteil im Bereich der Schamierlöcher in deren oberen, mittleren und unteren Position Positionierhilfen, z.B. transversal verlaufende Orientierungslinien, eingraviert oder angezeichnet, um die gewünschte Längeneinstellung der Beinteile besser und einfach kontrollieren zu können und somit die Größenanpassung vornehmen zu können.

Um eine Kippung des Kindes bei Rückenlage auf den mittelständigen Bug der Beckenplatte zu vermeiden, werden bevorzugter Weise auf der Rückseite derselben seitliche "Stabilisierungskeile", vorzugsweise aus einem oberflächendichten Schaumstoff und mit einem der eingebauten Vorhalte entsprechenden Keilwinkel von 35°, angebracht. Es ist dabei empfehlenswert, diese Vorhaltekeile hinten auf einer dritten formgerechten, jedoch planen, also nicht gebogenen Beckenplatte, z.B. mit Klettverschluss, verstellbar und verschiebbar, aber in Lage fixierbar aufzubringen, und diese dritte Beckenplatte mit dem gedoppelten Beckenteil am oberen Rand (unter dem Brustgurt) und in der Mittellinie zu vernähen oder zu verbinden. Der Klettverschluss wird vorzugsweise so angebracht, dass die Vorhaltekeile beiderseits um je etwa 3 cm nach oben außen ausgestellt werden können. Damit ist neben der Längenausstellung der Bandage durch die Achsverschiebung der Beinteile nach unten lateral auch eine Erweiterung des Beckenteils der Bandage nach oben-außen als Anpassung an das Wachstum des Kindes möglich. Alternativ können die Vorhaltekeile auch direkt an der dem Körper abgewandten Seite der gedoppelten und aufgebogenen Beckenplatte befestigbar sein.

Die beiden Beinteile sind vorzugsweise aus einer gleichartigen semiflexiblen Kunststoff-Folie (vorzugsweise Polyäthylen) von z.B. 1 bis 2 mm Dicke als ausreichend lange, breite Streifen hergestellt und dienen als Oberschenkelhalteschlaufen. Sie sind vorzugsweise symmetrisch gestaltet, werden jedoch seitenverkehrt verwendet. Dabei sind sie im oberen Bereich vorzugsweise so verrundet, dass ihre Drehbewegung zwischen den beiden Beckenplatten nicht behindert wird. Unter Berücksichtigung dessen, dass der Oberschenkel bei der Behandlung auf den Beinteilen quer aufliegt, muss die der Hüftkopflage entsprechende Achsperforation der Beinteile auf denselben aus der Längsachse derselben nach medial (nach innen) versetzt sein.

Um dem Längenwachstum des quer aufliegenden Oberschenkels Rechnung zu tragen, verbreitet sich das ausstellbare Beinteil nach oben vorzugsweise etwas, was vorzugsweise durch eine insgesamt konische Gestaltung des Beinteils erreicht wird. Der nach unten verschmälert auslaufende Teil des Beinteils kommt nach dem Anlegen der Bandage durch die Umschlingung des Oberschenkels von hinten unten nach vorne oben in den Bauchbereich. Am distalen Ende der Beinteile kann z.B. ein Klettverschlussstreifen oder eine andere Befestigung wie Knöpfe und Knopflöcher angenäht werden, welche eine Befestigung der hochgeschlagenen Beinteile vorne am Rumpfgurt der Bandage ermöglicht.

Im oberen Bereich der Beinteile soll vorzugsweise eine Beinteilwinkelanzeige, z.B. eine mit dem Ausgangspunkt des Scharnier-Bohrloches transversal absteigende eingravierte Winkelgraduierung vorgesehen sein, welche z.B. in 10°-Stufen von 0 bis 50° reicht. Die Winkellinien sind vorzugsweise am Ende mit den Zahlen der Grade zu bezeichnen. Um die Skala besser erkennbar zu machen, kann die vordere Beckenfolie durch ein entsprechendes Winkelskalenbeobachtungsloch durchbrochen sein.

Durch Drehung der Beinteile um die Scharnierachse kann dann bei Kongruenz der Winkellinien der Beinteile mit z.B. den durch das Scharnier-Schlitzloch des Beckenteiles verlaufenden Querlinien die gewünschte Beugestellung der Oberschenkel und damit der Hüftgelenke genau eingestellt werden.

Hier handelt es sich um Beugungsgrade über der Horizontalen, wie sie bei einfacheren Spreizhosen nicht eingestellt werden können. Die Therapie kann damit nach den individuellen Hüftverhältnissen, auch nach Bedarf asymmetrisch, d.h. unterschiedlich für beide Hüften, präzisiert werden. Die 0°-Einstellung als Bezugsbasis entspricht also bereits einer Hüftbeugung (gegenüber der Beinstreckstellung) von 90°.

Gemäß den bekannten muskeldynamischen Behandlungsprinzipien muss die Beugung um so steiler sein, je weiter die Hüfte auf der Basis der Dysplasie in Subluxation oder Luxation disloziert ist.

Aufgrund klinischer Erfahrungen sind für die entsprechenden Winkelgrade bezüglich der Horizontalen bzw. 90°-Hüftanbeugung folgende Einstellungen zu empfehlen, die als Kurzbezeichnungen mit eingraviert werden könnten:
- 0°/N: "Neutralstellung" für "mit zu behandelnde" gesunde kontralaterale Hüften.
- 10°/P: Prophylaktische Behandlung von Grenzfällen und auslaufende Nachsorge-Behandlung.
- 20°/D Dysplasie ohne Dislokation.
- 30°/L1: Beginnende Luxation.
- 50°/L2: Vollständige Luxation.

Die Befestigungsvorrichtung zur Befestigung zwischen dem Beckenteil und den Beinteilen erfolgt vorzugsweise über beiderseitige Löcher und eine dazwischen liegende Achsschraube. Wird eine solche Achsschraube verwendet, so handelt es sich vorzugsweise um eine metallische oder Kunststoff-Kompressionsschraube mit oder ohne hinterdrehtem Hals mit einem üblichen Schraubendrehereinsatz, z.B. halber Querschlitz, die vorzugsweise von vorne nach hinten durch die Achslöcher eingesteckt und auf der Rückseite mit einer drehsicheren, z.B. geriffelten oder an den Rändern gezackten, Mutter festgezogen wird. Zur Vermeidung eines Verlustes der Mutter bzw. Schraube bei Schraubenlockerung können die Endgewinde der Schraube nach der Mutterapplikation eine Verquetschung erfahren.

Um die Schraubenkompression auf eine größere Fläche zu verteilen, ist es empfehlenswert, dass unter dem Schraubenkopf und unter der Mutter je eine Beilagscheibe eingefügt wird.

Aus Festigkeitsgründen empfiehlt sich für die gesamte Achsverbindung die Verwendung von Metall, vorzugsweise rostfreien Stahl. Es ist aber auch hoch fester Kunststoff denkbar.

Alternativ kann als Befestigungseinrichtung ein in den Langlöchern geführter Clip, z.B. aus Kunststoff, verwendet werden. Da dieser keine sichere Lagepositionierung der Beinteile zum Beckenteil vorsieht, kann entweder durch geeignete Formgebung des Clipschafts, z.B. mit polygonförmigem Querschnitt, und entsprechender Randgestaltung des Langlochs oder durch vom Langloch getrennte, zusätzliche Sicherungsmittel, z.B. weitere, zusätzliche Stanzlöcher in dem Beckenteil und den Beinteilen, die den häufigsten Winkelpositionen und Längenwachstumspositionen entsprechen, mit einem zweiten Clip oder Klettmittel die Festlegung der Beinteile zum Beckenteil für die Benutzung erfolgen. Die stufenlose Verstellbarkeit geht dabei jedoch ggf. verloren.

Neben der Sicherung der Beinteile gegenüber dem Beckenteil durch die Befestigungseinrichtung, vorzugsweise in der Gestalt von Löchern und der Schraube, können zusätzliche Maßnahmen, welche ein Rutschen der Teile gegenseitig verhindern, vorgesehen werden. Beispielsweise können die Beinteile dazwischen aufnehmenden Beckenteile ebenso wie die Beinteile selbst durch aufgerauhte Kunststoffwerkstoffe gestaltet werden, die von sich aus aneinander haften und nicht leicht verrutschen, oder es können beidseitige Kletteinrichtungen vorgesehen werden.

Das vorstehend beschriebene Grundgerüst der Dysplasie-Bandage ist vorzugsweise auf der vorderen Körperseite, weiter vorzugsweise nur auf dem Beckenteil, mit einer z.B. etwa 1 cm dicken Polsterschicht, z.B. Moosgummi bzw. Schaumstoff mit porendichter Oberfläche, versehen. Dadurch kann eine Urindurchtränkung vermieden werden und Reinigungswaschung ermöglicht werden. Die Befestigung der Polsterschicht auf den Polyäthylenfolien erfolgt vorzugsweise nur am Oberrand des Beckenteils mit Naht, so dass die Polsterschicht von unten nach oben hochgeklappt werden kann und somit die Befestigungseinrichtung z.B. Achsschrauben und die Winkelskala, leicht sichtbar und zugänglich zu machen sind.

Darüber hinaus ist es angebracht, die Ränder der Polyäthylenfolien der Beinteile mit einer dünnen flexiblen Polsterumrandung mit wasserfestem Überzug zu umfassen und mit einer Steppnaht zu befestigen, um den Tragekomfort zu erhöhen.

Weiterhin weist die Bandage vorzugsweise ein Gurt-Trägersystem zu ihrer Befestigung am Patientenkörper auf.

Am oberen Rand des Beckenteils wird dazu z.B. hinten querverlaufend ein kräftiger halbsteifer Textilgurt von ca. 3-4 cm Breite befestigt, der seitlich um den Körper auf die Bauchseite herumreicht und dort mit Klettverschluss oder anderer Verschlusseinrichtung in variabler Umfangsweite einfach verschlossen werden kann. Am Vorderteil des verschlossenen Rumpfgurtes werden dann die um die Oberschenkel von hinten unten nach vorne oben herumgeschlungenen Beinteile (Oberschenkelhalteschlaufen) mit ihren Klettverschlussenden an entsprechender Stelle variabel befestigt.

Hinten ist am Rumpfgurt auf dem Beckenteil ein Schultergurt-Trägersystem befestigt, so dass sich die beiden Trägergurte z.B. am Rücken überkreuzen, über die gegenüberliegende Schulter nach vorne verlaufen und dort ungekreuzt mit längenverstellbaren Schnappschnallen an entsprechenden Aufnahmebuchsen des Rumpfgurtes befestigt werden können. Die Schulterträger werden vorzugsweise aus undehnbarem Textilband gefertigt und müssen im Auflagebereich der Schulter ausreichend gepolstert sein, vorzugsweise durch eine zirkulär aufgesteckte verschiebbare Polstermanschette.

Vorzugsweise wird die gesamte Hüftdysplasie-Bandage mit einem Textilschutzüberzug überzogen. Dieser ist vorzugsweise wasch- und dehnbar und hosenartig gestaltet und entspricht der Gesamtform der Bandage unter Berücksichtigung ihrer Ausstellbarkeit, so dass er alle Formen der Bandage aufnehmen kann.

Dieser Überzug soll vorzugsweise wie eine Hose oben sowie an den Enden der Beinteile unten offen sein, damit er auch hosenartig von unten nach oben über die Bandage gezogen werden kann.

Der Überzug soll am oberen Beckenteilrand leicht abnehmbar befestigt werden (vorzugsweise mit Klettverschluss oder Halteschlaufen, Knöpfen bzw. Druckknöpfen), um ein leichtes An- und Abziehen zum Waschen des Überzuges zu ermöglichen.

Nachfolgend wird die Erfindung rein beispielhaft anhand der beigefügten Figuren beschrieben, in denen:
- Fig. 1: die wesentlichen Einzelteile einer erfindungsgemäßen Hüftdysplasie-Bandage im demontierten Zustand zeigt;
- Fig. 2: schematisch die wesentlichen Einzelteile der erfindungsgemäßen Hüftdysplasie-Bandage im montierten Zustand zeigt;
- Fig. 3: das Beckenteil der Hüftdysplasie-Bandage aus Fig. 1 und 2 zeigt;
- Fig. 4: eine Querschnittsansicht durch das Beckenteil aus Fig. 3 im cranialen Beckenteil zeigt;
- Fig. 5: eine Querschnittsansicht durch das Beckenteil in Höhe der Befestigungseinrichtung der Beinteile zeigt;
- Fig. 6: ein Beinteil der erfindungsgemäßen Hüftdysplasie-Bandage zeigt;
- Fig. 7: eine Ansicht zum Erläutern der Abhängigkeit des Oberschenkelauflagewinkels von der Abspreizstellung der Beinteile ist;
- Fig. 8: die erfindungsgemäße Hüftdysplasie-Bandage in einem am Säugling angelegten Zustand zeigt;
- Fig. 9: die Verstellmöglichkeiten bei Größenwachstum des Säuglings erläutert; und
- Fig. 10: eine Ansicht der angelegten Bandage von unten mit Vorhalte der Oberschenkel ist.

Die in den Figuren dargestellte Ausführungsform der erfindungsgemäßen Hüftdysplasie-Bandage 10 enthält im Wesentlichen ein Beckenteil 12 sowie ein rechtes und ein linkes Beinteil 14, 16. Die Angaben oben, unten, rechts, links in dieser Beschreibung beziehen sich dabei jeweils auf den Zustand der angelegten Hüftdysplasie-Bandage 10 am Säugling, wobei mit "vorne" die dem Körper zugewandte Seite und mit "hinten" die dem Körper abgewandte Seite bezeichnet ist.

Das Beckenteil 12, das rechte Beinteil 14 und das linke Beinteil 16 sind über eine Befestigungseinrichtung 20, die später genauer erläutert wird, aneinander befestigbar und in ihrer Lage zueinander fixierbar. Dies bedeutet, dass das erfindungsgemäße Beckenteil 12 und das rechte Beinteil 14 sowie das linke Beinteil 16 als voneinander separierbare Elemente gestaltet sind, die jedoch für den Gebrauch in verschiedenen Lagen je nach Bedarf zueinander festgelegt werden können und im Gebrauch mittels der Befestigungsvorrichtung 20 in diesen Lagen in verschiedenen Abspreiz-Winkelstellungen festgelegt werden können.

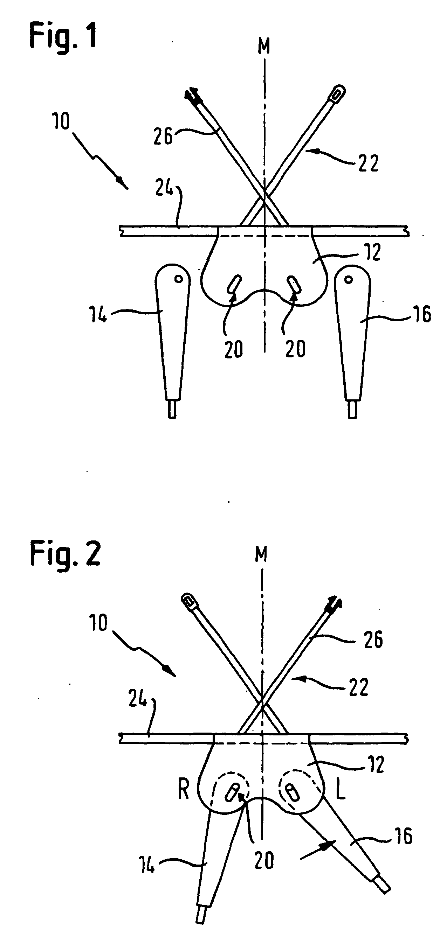

Wie insbesondere aus Fig. 2 entnehmbar ist, können das rechte Beinteil 14 und das linke Beinteil 16 (die Richtungen rechts und links sind in den Figuren mit "R" bzw. mit "L" bezeichnet) auch bezüglich einer Mittelachse M zueinander asymmetrisch am Beckenteil 12 angebracht werden.

Wie aus Fig. 1 und 2 entnehmbar ist, ist das Beckenteil 12 ferner mit einem Gurt-Trägersystem 22 versehen, das insbesondere einen quer verlaufenden, um die Hüfte des Säuglings legbaren Hüft- bzw. Rumpfgurt 24, beispielsweise ausgeführt als halbsteifer Textilgurt mit ca. 3 bis 4 cm Breite, und sich von dem als Textilgurt ausgeführten Rumpfgurt 24 aus erstreckende Hosenträgergurte bzw. Schultergurte 26 enthält, die über die Schultern des Säuglings zu legen sind und an ihren beiden Enden mit dem quer verlaufenden Hüftgurt 24 zu verbinden sind.

Beim Anlegen der Hüftdysplasie-Bandage 10 am Säugling wird somit der Säugling mit seinem Gesäß auf das Beckenteil 12 gelegt, der Rumpfgurt 24 wird um den Bauch des Säuglings geschlungen und beispielsweise über nicht dargestellte Klettverschlussmittel locker bis verhältnismäßig eng anliegend um den Rumpf verschlossen. Anschließend werden die Schultergurte 26 so über die Schultern des Säuglings gelegt, dass sich die beiden Schultergurte 26 am Rücken des Säuglings überkreuzen und über die gegenüberliegende Schulter nach vorne verlaufen. Dort können sie ungekreuzt, beispielsweise durch Schnalleneinrichtungen, die am Rumpfgurt 24 befestigt sind, verschnappt werden. Vorzugsweise ist im Bereich der Schultergurte 26 zusätzlich jeweils eine Längenverstelleinrichtung vorgesehen, die wahlweise an den Schnappschnallen am Hüftgurt 24 oder den Schultergurten 26 an sich vorgesehen sein kann. Die Schultergurte 26 sind vorzugsweise aus undehnbarem Textilband gefertigt. Um den Komfort zu erhöhen, können sie im Auflagebereich auf der Schulter mit einer Poltereinrichtung, beispielsweise in sie umhüllender Röhrenform, versehen sein (nicht dargestellt).

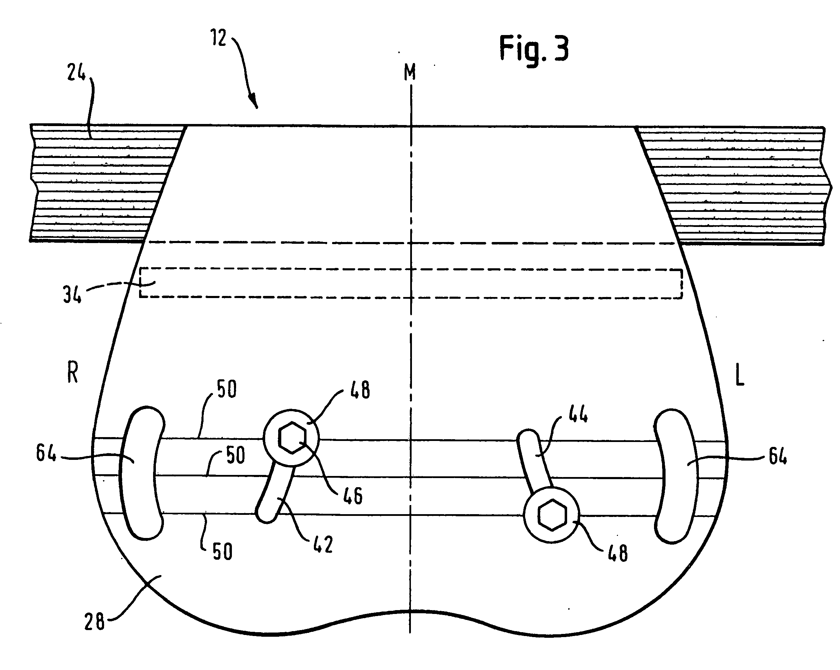

Das in Fig. 3 bis 5 genauer dargestellte Beckenteil 12 bildet in der Draufsicht, die in Fig. 3 dargestellt ist, im Wesentlichen die Form des kindlichen Beckens mit den Weichteilen nach und reicht von der Rumpftaille bis zu den Gesäßfalten. Dort ist das Beckenteil 12, insbesondere dessen Beckenplatte 28 abgerundet (unten in Fig. 3).

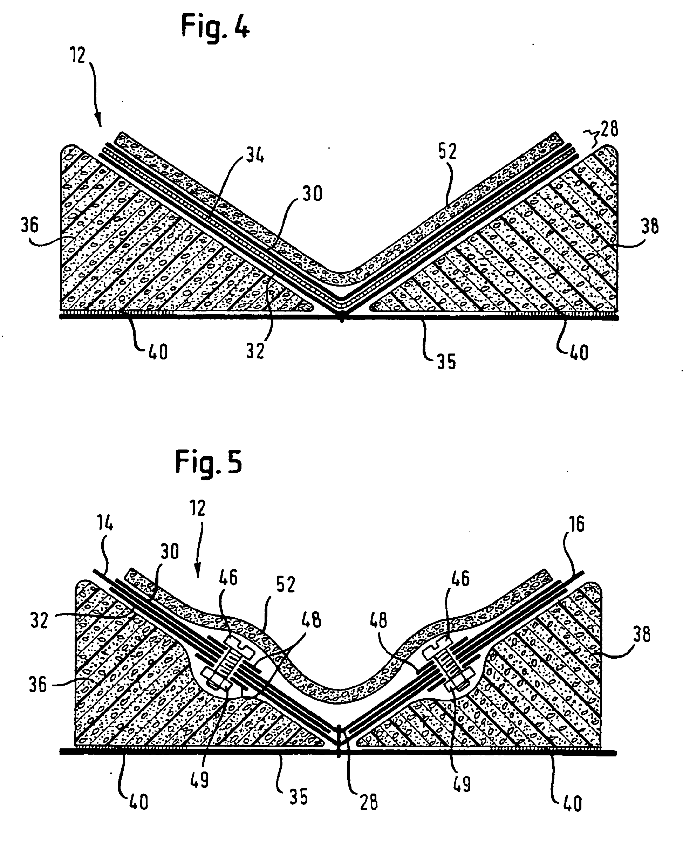

Die das Beckenteil 12 maßgeblich formende Beckenplatte 28 ist, wie am Besten aus Fig. 4 und 5 entnehmbar ist, aus zwei gleich geformten, vorzugsweise halbsteifen Kunststofffolienteilen 30, 32, beispielsweise aus Polyethylen mit 1 bis 2 mm Dicke, gebildet. Die vordere Folie 30 (entsprechend der dem Körper des Säuglings zugewandten Seite) und die hintere Folie 32 sind im oberen Taillenbereich der Beckenplatte 28, d.h. im Bereich der Taille des Säuglings, miteinander verbunden, beispielsweise durch Vernähen. Im latero-kaudalen Bereich bleiben sie hingegen unverbunden, so dass sie in Bezug aufeinander aufgeklappt werden können und die später zu beschreibenden Beinteile 14, 16 dazwischen eingeschoben werden können (siehe Fig. 5). Im Taillenbereich der Beckenplatte 28 ist der Rumpfgurt 24 angebracht (Fig. 3).

Wie aus Fig. 4 und 5 erkennbar ist, ist die Beckenplatte 28 nicht plan (eben) gestaltet, sondern bezüglich der Mittelachse M, die gleichzeitig Symmetrieachse der Beckenplatte 28 ist, aufgebogen, vorzugsweise um beispielsweise 35° beidseitig. Diese aufgebogene Stellung kann durch eine Bügelschiene 34, beispielsweise aus Metall, zuverlässig sichergestellt werden. Dazu ist die Bügelschiene 34, die einen Mittenwinkel von 110° einschließt, zwischen die vordere Folie 30 und die hintere Folie 32 im oberen Bereich des Beckenteils 12 eingebracht und damit z.B. durch Verkleben fest verbunden. Diese Bügelschiene 34 ist vorzugsweise als streifenförmiger Querbügel gestaltet und kann neben Metall auch aus steifem Kunststoff, wie beispielsweise PVC, oder ähnlichem gebildet sein.

Ferner sind zur Sicherung der Abspreiz-Vorhaltestellung neben der Biegung der Beckenplatte 28 und dem Einbringen der Bügelschiene 34 vorzugsweise zusätzlich jeweilige Keile 36, 38 an der Hüftdysplasie-Bandage 10, insbesondere deren Beckenteil 12, vorgesehen. Die Stützkeile 36, 38 liegen unter der hinteren Folie 32, d.h. sie schließen sich auf der zum Säugling abgewandten Seite daran an. Sie können entweder direkt an der hinteren Folie 36 befestigt sein, vorzugsweise über eine Verstelleinrichtung, welche eine seitliche Verschiebbarkeit der Keile 36, 38 in zumindest begrenztem Umfang erlaubt, oder sie sind, wie es in Fig. 4 und 5 gezeigt ist, zwischen eine weitere, dritte formgerechte, aber ebener, nicht aufgebogene Folie 35 der Beckenplatte 28 und die hintere Folie 32 eingebracht. Als Verstelleinrichtung 40 dient in diesem Fall eine Kletteinrichtung, die auf der dritten Folie 35, welche eine dorsale Abschlussplatte bildet, und den Keilen 36, 38 zur gegenseitigen Wechselwirkung vorgesehen ist. Die Keile 36, 38 sind dabei neben der in Fig. 4 und 5 gezeigten Position auch jeweils in einer diesbezüglich nach außen verschobenen Position (Rechts-Links-Richtung in Fig. 4 und 5) befestigbar, so dass sie beispielsweise jeweils um je 3 cm nach außen und leicht oben ausgestellt werden können, so dass eine Erweiterung des Beckenteils 12 der Hüftdysplasie-Bandage 10 nach oben-außen als Anpassung an das Wachstum des Kindes möglich ist.

Die Stützkeile 36, 38 sind beispielsweise aus einem oberflächendichten Schaumstoff und mit einem der eingebauten Vorhalte entsprechenden Keilwinkel von 35° gestaltet. Zur Lagesicherung ist in der dargestellten Ausführungsform gemäß Fig. 4 und 5 die dritte Folie 35 mit der Mittellinie M (Symmetrie-Linie) der vorderen Folie 30 und hinteren Folie 32 vernäht bzw. verbunden.

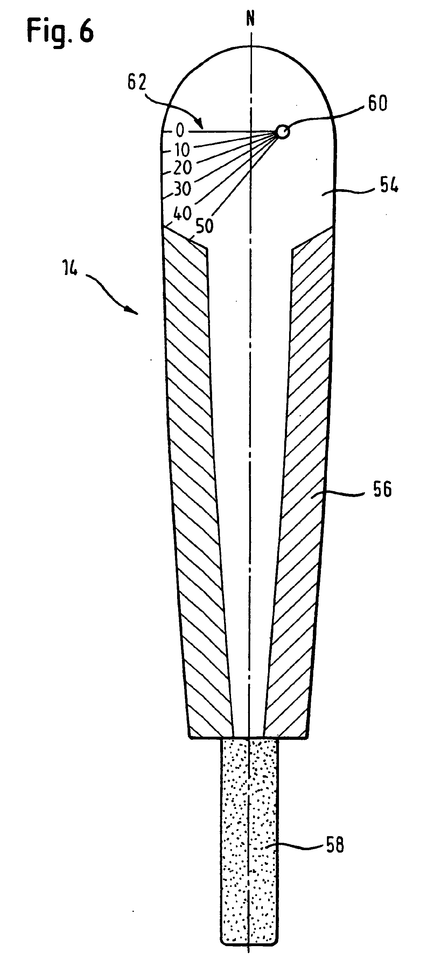

Die Beckenplatte 28 ist, wie aus Fig. 3 und 5 entnehmbar ist, mit Mitteln zur Befestigung der Beinteile (siehe Fig. 6) versehen. Dazu ist in die Beckenplatte 28 jeweils ein Langloch 42, 44 symmetrisch zur Mittelachse M eingebracht, durch das beispielsweise eine Schraube 46 (dargestellt in Fig. 5) geführt werden und mit Beilagscheiben 48 und einer Mutter 49 gesichert werden kann. Innerhalb des Langlochs 42, 44 kann die Schraube 46 im nicht angezogenen Zustand der Schraube verschoben werden.

In Fig. 3 ist für das rechte Bein (linke Seite in Fig. 3) die kürzeste Befestigungsposition für die Beinteile dargestellt, während für das linke Bein (rechte Seite in Fig. 3) die Position maximaler Beinteilverlängerungsstellung dargestellt ist. Durch die Gestaltung der Verstelleinrichtung in Form eines Langlochs 42, 44 und der Befestigung der Beinteile mittels der Schraubeneinrichtung 46, 48, 49 ist eine stufenlose Verstellung und Längenanpassung möglich. Alternativ können mehrere einzelne Löcher statt des durchgängigen Achslochs 42, 44 vorgesehen werden, welche jedoch bei fortschreitendem Wachstum des Kindes keine kontinuierliche, sondern eine stufenweise Verstellung der Schraubachse nach distal-lateral und damit eine Verlängerung der Beinteile ermöglichen. In der dargestellten Ausführungsform sind zur Vereinfachung der Einstellung der Länge der Beinteile Orientierungslinien 50 auf der Beckenplatte 28 vorgesehen, so dass der Therapeut einfach eine gewünschte Längenanpassung vornehmen kann. Beispielsweise können die Linien mit Angaben versehen sein, welche einen Rückschluss auf die effektive Beinteillänge der Hüftdysplasie-Bandage 10 zulassen.

Die Beinteile 14, 16 werden, wie es in Fig. 5 gezeigt ist, durch Anziehen der Schraubeneinrichtung 46, 48, 49 und Einbringen der Beinteil 14, 16 zwischen die vordere Folie 30 und die hintere Folie 32 der Beckenplatte 28 lage- und drehfest bezüglich des Beckenteils 12 befestigt.

Zum Verbessern des Komforts des Beckenteils 12 ist auf der der Körperseite zugewandten Seite der Beckenplatte 28 eine Polsterauflage 52 vorgesehen, die aus einem beispielsweise oberflächendichten, weichen Kunststoffschaummaterial gebildet ist und z.B. 1 cm dick ist. Die Polsterauflage 52 ist vorzugsweise ebenfalls nur am oberen Rand (Taillenbereich) mit der vorderen Folie 30 und der hinteren Folie 32 verbunden, so dass eine gute Zugänglichkeit zur Befestigungseinrichtung für die Beinteile 14, 16 gewährleistet ist.

Die Beinteile sind, wie es in Fig. 6 für das rechte Beinteil 16 dargestellt ist, aus einer semiflexiblen Kunststofffolie 54, beispielsweise Polyethylen, streifenförmig gebildet. Durch Umschlingung der Oberschenkel des Säuglings dienen sie als Oberschenkelhalteschlaufen. Das linke und das rechte Beinteil 16, 14 sind bezüglich der Mittelachse M der Bandage symmetrisch gefertigt. Die Asymmetrie der Hüftdysplasie-Bandage 10, die ggf. gewünscht ist, um unterschiedliche Fehlstellungen der beiderseitigen Hüften verschiedenartig korrigieren zu können, wird durch entsprechende Festlegung der Beinteile 14, 16 am Beckenteil 12 erreicht. Im Randbereich ist die die Beinteile bildende Folie 54 vorzugsweise mit einer Randpolsterung 56 versehen, um den Tragekomfort zu erhöhen. Die Randpolsterung 56 kann an der Folie 54 beispielsweise durch Steppnähte befestigt sein und einen wasserfesten Überzug enthalten. Am freien Ende der Beinteile 14, 16, d.h. dem Ende, das nicht am Beckenteil 12 befestigt wird, ist beispielsweise ein Klettband 58 als Verschlusseinrichtung und zum Verbinden mit dem Rumpfgurt 24 der Bandage 10 vorgesehen. Der Klettbereich 58 kann somit mit einem Gegenstück Klettbereich am Rumpfgurt 24 verharkt werden.

Die Beinteile 14, 16 weisen in ihrem oberen Bereich ferner ein Loch 60 auf, durch das die Schraube 46 geführt werden kann. Das Loch 60 ist bezüglich einer Mittellängsachse N der Beinteile 14, 16 versetzt und zwar nach innen bezüglich der Bandage 10. Die Gestaltung der Folie 54 für die Beinteile 14, 16 sowie des Bereichs der Befestigungsvorrichtung kann jedoch nach Bedarf modifiziert werden, wobei jedoch vorzugsweise sowohl eine Drehbarkeit der Beinteile 14, 16 bezüglich des Beckenteils 12 als auch eine Verschiebbarkeit zur Kompensation des Längenwachstums des Säuglings möglich sind.

Um die Winkelstellung der Beinteile bezüglich des Beckenteils 12 gut justieren zu können, ist am Beinteil vorzugsweise eine Winkelskala 62 angebracht, welche zusätzlich nach Bedarf Kurzbezeichnungen für die jeweiligen Hüftstellungen beinhalten kann (nicht dargestellt). Zur besseren Erkennbarkeit der Winkelskala 62 durch die vordere Folie 30 des Beckenteils 12 ist dieses beiderseits mit Winkelskalensichtlöchern 64 versehen.

Die Gesamtform der Beinteile 14, 16 ist konusförmig nach unten (in Richtung freies Ende) zulaufend, so dass dem Längenwachstum des quer aufliegenden Oberschenkels Rechnung getragen wird.

Vorzugsweise ist (nicht dargestellt) für die gesamte Hüftdysplasie-Bandage 10 ein Textilschutzüberzug vorgesehen, der hosenartig gestaltet ist, und der alle Winkelstellungen der Beinteile 14, 16 zum Beckenteil 12 sowie Längenänderungen aufnimmt, in den die aus Kunststoffmaterialien gebildete Hüftdysplasie-Bandage 10 eingeführt werden kann. Dieser Überzug, der leicht abnehmbar ist, kann somit bei Verschmutzung gewaschen werden und verbessert das Erscheinungsbild der Hüftdysplasie-Bandage 10.

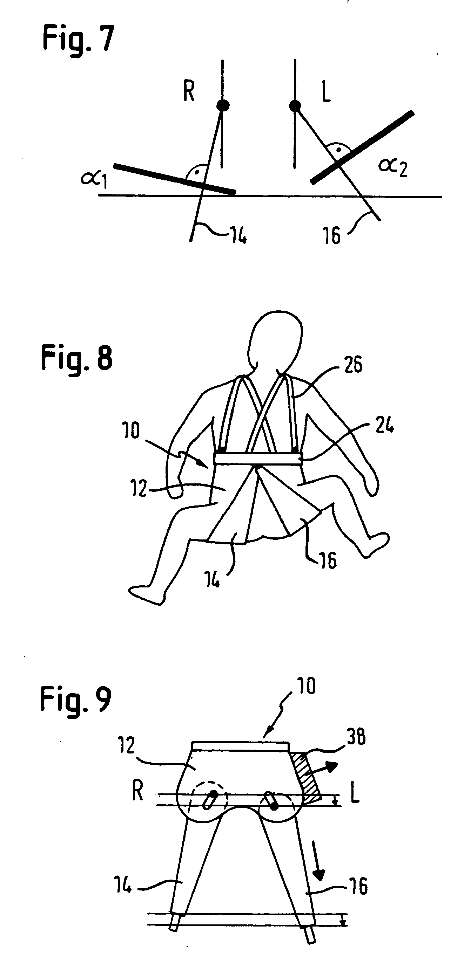

Zur Anwendung der erfindungsgemäßen Hüftdysplasie-Bandage wird zunächst vom Therapeuten der erforderliche Beugewinkel bestimmt. Wie in Fig. 7 zu erkennen ist, ist der Oberschenkelauflagewinkel α abhängig von der Abspreizstellung der Beinteile 14, 16, die schematisch als Striche angedeutet sind. Bei geringerer Abspreizung der Beinteile, wie es für die rechte Seite R dargestellt ist, resultiert ein kleiner Beugewinkel α₁, während bei stärkerer Abspreizung des Beinteils 16 (linke Seite) eine starke Oberschenkel- und damit Hüftgelenksanbeugung mit großem Abspreizwinkel α₂ resultiert.

Nach Bestimmung des erforderlichen Abspreizwinkels α kann der Therapeut durch Verstellen der Winkelstellung der Beinteile 14, 16 jeweils individuell bezüglich des Beckenteils 12 die Abspreizstellung justieren. Gleichzeitig kann er durch Verschieben der Befestigungsvorrichtung 20 sowie ggf. durch Verschieben der Keile 36, 38 nach außen zur Kompensation des Größenwachstums des Säuglings eine Größenanpassung vornehmen, wie es schematisch in Fig. 9 dargestellt ist.

Fig. 8 zeigt die angelegte Hüftdysplasie-Bandage 10 mit seitenverschiedener, den Therapieerfordernissen entsprechender Oberschenkelbeugung und damit Hüftgelenksbeugung. Nach Bedarf kann die Oberschenkelanbeugung und Hüftgelenksanbeugung auch während der Therapie problemlos nachjustiert und/oder korrigiert werden, indem einfach durch Lockern der Schraubeneinrichtung und Verdrehen von einem oder beiden Beinteilen 14, 16 bezüglich des Beckenteils 12 die Winkelstellung korrigiert wird. Somit kann die erfindungsgemäße Hüftdysplasie-Bandage "mitwachsen" und den Therapieerfordernissen angepasst werden, so dass z.B. mit Hüftdysplasie-Bandagen 10 in zwei Größen das Wachstum des Säuglings von 0 Monaten bis 12 Monaten kompensiert werden kann.

Fig. 9 veranschaulicht die Justiermöglichkeiten der erfindungsgemäßen Hüftdysplasie-Bandage 10 zur Anpassung an das Größenwachstum des Säuglings. Für die rechte Seite R ist eine kurze Ausgangsstellung des Beinteils 14 dargestellt, d.h. das Beinteil 14 ist am Beckenteil 12 in der am weitesten oben liegenden Endanschlagsposition im Langloch befestigt. Gleichzeitig ist das zugehörige Keilelement 36 in der am weitesten innen liegenden Position, so dass es über die Folien 30, 32 seitlich nicht hervorsteht. Im Gegensatz dazu ist für das linke Beinteil 16 die am weitesten verlängerte Position dargestellt, die durch Verschieben der Achsschraube 46 im Langloch 42 an den unteren Anschlag (Langlochrand) erzielt wird. Gleichzeitig kann das Beckenteil 12 in latero-cranialer Richtung durch seitliche Ausstellung des Stützkeils 38 vergrößert werden, so dass die Hüftdysplasie-Bandage 10 komfortable zu tragen bleibt. Anders als die Anpassung an die Hüftdysplasie des Säuglings, die in Verbindung mit Fig. 7 und 8 erläutert ist und asymmetrisch sein kann, wird die Längenanpassung gemäß Fig. 9 anders in Fig. 9 dargestellt, vorzugsweise symmetrisch durchgeführt.

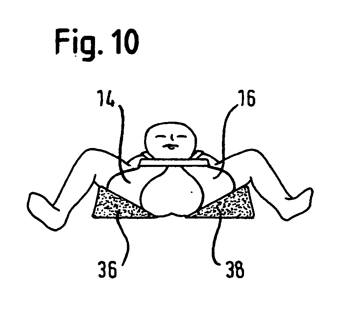

Fig. 10 zeigt schließlich eine Ansicht der angelegten Bandage von unten, aus der die Vorhaltestellung der Oberschenkel des Säuglings mittels des angestellten Beckenteils 12 und der Stützkeile 36, 38 gut erkennbar ist.

Vorzugsweise werden alle Elemente der Hüftdysplasie-Bandage 10 ausschließlich aus körperverträglichen, allergenfreien Kunststoffen hergestellt. Die Hüftdysplasie-Bandage kann in einer oder mehreren Größen vorgehalten werden, so dass sie das Säuglingsalter von der Geburt bis etwa zum Ablauf des ersten Lebensjahres abdecken kann. Beispielsweise können zwei verschiedene Größen jeweils mit Ausstellbarkeit der Beinteile 14, 16 vorgesehen werden.

Anstatt bei der beschriebenen Ausführungsform Langlöcher 44 an dem Beckenteil 12 vorzusehen, können die Langlöcher auch an dem Beinteil 14 bzw. 16 vorgesehen sein und am Beckenteil 12 einfache rundliche Löcher vorgesehen werden. Die Winkelskala an den Beinteilen 14, 16 bzw. nach einer alternativen Ausführungsform am Beckenteil 12 ist vorzugsweise für Winkel zwischen 0° und 50° ausgelegt, weiter vorzugsweise mit 10°-Stufen.

### Bezugszeichenliste

- 10: Hüftdysplasie-Bandage
- 12: Beckenteil
- 14: Beinteil
- 16: Beinteil
- 20: Befestigungseinrichtung
- 22: Gurt-Trägersystem
- 24: Rumpfgurt
- 26: Hosenträgergurtsystem
- 28: Beckenplatte
- 30: Folienteil
- 32: Folienteil
- 34: Bügelschiene
- 36: Stützkeil
- 38: Stützkeil
- 40: Verstelleinrichtung
- 42: Langloch
- 44: Langloch
- 46: Schraube
- 48: Scheibe
- 50: Orientierungslinie
- 52: Polsterauflage
- 54: Folie
- 56: Randpolsterung
- 58: Klettband
- 60: Loch
- 62: Winkelskala
- 64: Winkelskalensichtloch

## Patentansprüche

1. Hüftdysplasie-Bandagenorthese (10) zur Frühbehandlung einer Hüftdysplasie bei Neugeborenen/Säuglingen, enthaltend ein Beckenteil (12) und davon getrennte Beinteile (14, 16) zur Umschlingung der Oberschenkel, wobei die Beinteile (14, 16) mit dem Beckenteil (12) mittels einer Befestigungseinrichtung (20) derart lösbar verbindbar sind, dass ihre Lage bezüglich des Beckenteils (12) in verschiedenen Abspreizstellungen und/oder Längenwachstumsanpassstellungen festlegbar ist, **dadurch gekennzeichnet, daß** die Beinteile (14, 16) streifenförmig sind, und daß das Beckenteil (12) eine Beckenplatte (28) mit zwei bereichsweise aneinander befestigten Kunststofffolien (30, 32) enthält, wobei die Beinteile (14, 16) aus Kunststofffolie gebildet sind und zwischen die Folien (30, 32) der Beckenplatte einschiebbar und fixierbar sind.

2. Hüftdysplasie-Bandagenorthese (10) nach Anspruch 1, wobei die Kunststofffolien dem Beckenumriss des Neugeborenen/Säuglings entsprechen.

3. Hüftdysplasie-Bandagenorthese (10) nach Anspruch 1 oder 2, **dadurch gekennzeichnet, dass** die Kunststofffolien (30, 32) Polyethylenfolien sind.

4. Hüftdysplasie-Bandagenorthese (10) nach einem der vorhergehenden Ansprüche, **dadurch gekennzeichnet, dass** die Befestigungsvorrichtung (20) Löcher (44) am Beckenteil (12) sowie Löcher (60) an den Beinteilen (14, 16) und ein Befestigungsmittel zur Fixierung der Beinteile (14, 16) bezüglich des Beckenteils (12) in ihrer Lage enthält.

5. Hüftdysplasie-Bandagenorthese (10) nach Anspruch 4, **dadurch gekennzeichnet, dass** die Löcher (44) in dem Beckenteil (12) als Langlöcher gestaltet sind, die schräg nach unten außen verlaufen, um eine Längenausstellung und Breitenausstellung der Beinteile (14, 16) bezüglich des Beckenteils (12) zu ermöglichen.

6. Hüftdysplasie-Bandagenorthese (10) nach Anspruch 4 oder 5, **dadurch gekennzeichnet, dass** das Befestigungsmittel eine durch die Löcher (44, 60) führbare Schraubeneinrichtung (46) ist.

7. Hüftdysplasie-Bandagenorthese (10) nach einem der vorhergehenden Ansprüche, **dadurch gekennzeichnet, dass** das Beckenteil (12) aus einer Beckenplatte (28) gebildet ist, die mittlings bugartig nach hinten gebogen ist, so dass die Beckenteilhälften in einem Querschnitt durch das Beckenteil, der senkrecht zu einer Symmetrieachse (M) der Beckenplatte ist, gegen eine ebene Beckenplatte um einen Vorhaltewinkel von jeweils 35° gedreht sind.

8. Hüftdysplasie-Bandagenorthese (10) nach einem der vorhergehenden Ansprüche, **dadurch gekennzeichnet, dass** das Beckenteil (12) aus drei gleich geformten Folienteilen (30, 32, 35) gebildet ist, wobei das dem Körper abgewandteste hinterste Folienteil (35) planar ist und mit den anderen Folienteilen (30, 32) entlang der Mittelachse verbunden ist.

9. Hüftdysplasie-Bandagenorthese (10) nach einem der vorhergehenden Ansprüche, **dadurch gekennzeichnet, dass** das Beckenteil (12) am oberen Rand mit einem Rumpfgurt (24) versehen ist, der um den Rumpf des Kindes schlingbar ist und an der Körpervorderseite mit einer Verschlusseinrichtung variabel schließbar ist.

10. Hüftdysplasie-Bandagenorthese (10) nach einem der vorhergehenden Ansprüche, **dadurch gekennzeichnet, dass** ein Hosenträgergurtsystem (26) vorgesehen ist, das am Beckenteil (12) befestigt ist.

## Claims

1. Bandage orthosis for hip dysplasia (10) for early treating hip dysplasia of new-borns/infants, comprising a pelvis member (12) and two leg members (14, 16) separated thereof for embracing thighs, wherein the leg members (14, 16) are removably connectable to the pelvis member (12) by means of a fixation device (20) such that the position thereof with respect to the pelvis member (12) is fixable at different abduction and/or length growth adaption positions, **characterized in that** the leg members (14, 16) are strip-shaped and the pelvis member (12) comprises a pelvis plate (28) having two plastic foils (30, 32) locally attached to each other, wherein the leg members (14, 16) are formed of plastic foil and are insertable between the foils (30, 32) of the pelvis plate and fixable.

2. Bandage orthosis for hip dysplasia (10) according to claim 1, wherein the plastic foils match with the profile of the pelvis of the newborn/infant.

3. Bandage orthosis for hip dysplasia (10) according to claim 1 or 2, **characterized in that** the plastic foils (30, 32) are polyethylene foils.

4. Bandage orthosis for hip dysplasia (10) according to one of the preceding claims, **characterized in that** the fixation device (20) comprises holes (44) on the pelvis member (12) and holes (60) on the leg members (14, 16) and a fixation means for fixing the leg portions (14) at their position with respect to the pelvis member (12).

5. Bandage orthosis for hip dysplasia (10) according to claim 4, **characterized in that** the holes (44) in the pelvis member (12) are shaped as elongated holes extending transversely downwards to an outside to allow a length displacement and a width displacement of the leg members (14, 16) with respect to the pelvis member (12).

6. Bandage orthosis for hip dysplasia (10) according to claim 4 or 5, **characterized in that** the fixation means is a screw device (46) insertable through the holes (44, 60).

7. Bandage orthosis for hip dysplasia (10) according to one of the preceding claims, **characterized in that** the pelvis member (12) is formed from a pelvis plate (28) being bent bow-like backwards in the middle such that the pelvis member halves are respectively turned relative to a plane pelvis plate in a lead angle of 35° in a cross section through the pelvis member, which cross-section is perpendicular to an axis of symmetry (M) of the pelvis plate.

8. Bandage orthosis for hip dysplasia (10) according to one of the preceding claims, **characterized in that** the pelvis member (12) is formed from three equally formed foil members (30, 32, 35), wherein the rearmost foil member (35) being farthest from the body is planar and is connected to the other foil members (30, 32) along the middle axis.

9. Bandage orthosis for hip dysplasia (10) according to one of the preceding claims, **characterized in that** the pelvis member (12) is provided with a torso belt (24) on the upper edge being embraceable around the torso of the child and being lockable by means of a locking device at the torso front.

10. Bandage orthosis for hip dysplasia (10) according to one of the preceding claims, **characterized in that** a suspenders belt system (26) is provided which is attached to the pelvis member (12).

## Revendications

1. Orthèse en bandage pour dysplasie de la hanche (10) pour le traitement précoce d'une dysplasie de la hanche chez les nouveau-nés/nourrissons, contenant une partie ventrale (12) et des parties destinées aux jambes séparées de celle-ci (14, 16) pour l'enserrement de la cuisse, les parties destinées aux jambes (14, 16) pouvant être liées à la partie ventrale (12) au moyen d'un dispositif de fixation (20) mais d'une manière détachable telle que son état par rapport à la partie ventrale (12) est fixable dans différentes positions d'extension latérale et/ou positions s'adaptant à la croissance en longueur, **caractérisée en ce que** les parties destinées aux jambes (14, 16) sont en forme de bandes et **en ce que** la partie ventrale (12) contient une plaque ventrale (28) avec deux feuilles de matière synthétique (30, 32) fixées l'une à l'autre par zones, les parties destinées aux jambes (14, 16) étant formées en feuille de matière synthétique et pouvant être glissées et fixées entre les feuilles (30, 32) de la plaque ventrale.

2. Orthèse en bandage pour dysplasie de la hanche (10) selon la revendication 1, dans laquelle les feuilles de matière synthétique correspondent au contour de l'os iliaque du nouveau-né/nourrisson.

3. Orthèse en bandage pour dysplasie de la hanche (10) selon la revendication 1 ou 2, **caractérisée en ce que** les feuilles de matière synthétique (30, 32) sont des feuilles en polyéthylène.

4. Orthèse en bandage pour dysplasie de la hanche (10) selon l'une des revendications précédentes, **caractérisée en ce que** le dispositif de fixation (20) contient des trous (44) sur la partie ventrale (12) ainsi que des trous (60) sur les parties destinées aux jambes (14, 16) et un moyen de fixation pour la fixation des parties destinées aux jambes (14, 16) en rapport avec la partie ventrale (12) dans son état.

5. Orthèse en bandage pour dysplasie de la hanche (10) selon la revendication 4, **caractérisée en ce que** les trous (44) dans la partie ventrale (12) sont configurés comme des trous oblongs qui sont orientés en oblique vers le bas et l'extérieur pour permettre un ajustement longitudinal et un ajustement en largeur des parties destinées aux jambes (14, 16) par rapport à la partie ventrale (12).

6. Orthèse en bandage pour dysplasie de la hanche (10) selon la revendication 4 ou 5, **caractérisée en ce que** le moyen de fixation est un dispositif à visser (46) qu'on conduit au travers des trous (40, 60).

7. Orthèse en bandage pour dysplasie de la hanche (10) selon l'une des revendications précédentes, **caractérisée en ce que** la partie ventrale (12) est formée d'une plaque ventrale (28) qui est pliée au centre en courbure vers l'arrière, de sorte que les moitiés de la partie ventrale tournent, en coupe transversale à travers la partie ventrale, qui est à la verticale d'un axe de symétrie (M) de la plaque ventrale, contre une plaque ventrale plate d'un angle de jet de chaque fois 35°.

8. Orthèse en bandage pour dysplasie de la hanche (10) selon l'une des revendications précédentes, **caractérisée en ce que** la partie ventrale (12) est formée de trois parties en feuille de forme identique (30, 32, 35), la partie en feuille la plus arrière opposée au corps (35) étant plane et reliée avec les autres parties en feuille (30, 32) le long de l'axe central.

9. Orthèse en bandage pour dysplasie de la hanche (10) selon l'une des revendications précédentes, **caractérisée en ce que** la partie ventrale (12) est munie, sur le bord supérieur, d'une ceinture de tronc (24) dont on peut ceindre le tronc de l'enfant et qu'on peut fermer de façons variables sur l'avant du corps avec un dispositif de fermeture.

10. Orthèse en bandage pour dysplasie de la hanche (10) selon l'une des revendications précédentes, **caractérisée en ce qu'**un système de ceinture de port de pantalon (26) est prévu, qui est fixé sur la partie ventrale (12).
